# EUROPEAN PATENT APPLICATION

(11) **EP 1 031 625 A2**
(43) Date of publication of application: **30.08.2000**
(21) Application number: 00100081.9
(22) Date of filing: 05.01.2000
(51) Int. Cl.: C12N 9/02, C12N 1/00

(54) **Method for enhancing activity to regenerate electron acceptor for oxidoreductase in microorganism capable of producing said oxidoreductase, and use of microorganism prepared by said method**

(30) Priority: 07.01.1999 JP 203999
(71) Applicant: DAICEL CHEMICAL INDUSTRIES, Ltd., Sakai-shi, Osaka 590-0905 (JP)
(72) Inventor: Yamamoto, Hiroaki, Tsukuba-shi, Ibaraki 305-0047 (JP)
(74) Representative: VOSSIUS & PARTNER

(57) **Abstract**

A method for enhancing activity to regenerate electron acceptors for oxidoreductases in microorganisms capable of producing the oxidoreductases is provided. A method for producing useful substances such as optically active alcohols using the microorganisms is also provided. Microorganisms having high activity to regenerate the oxidized form of an electron acceptor can be obtained by culturing microorganisms capable of producing oxidoreductases in a culture medium with low concentration of dissolved oxygen.

## Description

### FIELD OF THE INVENTION

The present invention relates to a method for enhancing activity to regenerate electron acceptors for oxidoreductases in microorganisms capable of producing said oxidoreductases, microorganisms prepared by said method, and a method for producing useful substances using said microoragnisms.

### BACKGROUND OF THE INVENTION

Oxidoreductases are a group of enzymes classified into E.C.1. in accordance with the Enzyme Nomenclature - Recommendations (1978) of the Nomenclature Committee of the International Union of Biochemistry, Academic Press (1979), and utilizes a carbon-alcohol bond, an aldehyde group, a carbon-carbon bond, a carbon-nitrogen bond, etc. as electron donors, and nicotinamide adenine dinucleotide (NAD⁺), nicotinamide adenine dinucleotide phosphate (NADP⁺), cytochromes, molecular oxygen, quinones as electron acceptors.

These enzymes have high substrate specificity, stereoselectivity and reactivity, and useful substances such as optically active alcohols, aldehydes, ketones, amino acids, amino alcohols, organic acids, and fatty acids are manufactured using microorganisms containing these enzymes, disrupted microbial cells, partially purified enzymes, etc.

For producing useful substances using oxidoreductases, it is necessary to prepare microoragnisms having not only high oxidoreductase activity but also high activity to regenerate electron acceptors for redox. For example, in the reaction where one of stereoisomers of racemic alcohol is stereoselectively oxidized to recover the remaining optically active alcohol using alcohol dehydrogenase, the electron acceptor NAD⁺ is reduced to NADH as the oxidation reaction proceeds. It is thus necessary to oxidize NADH to NAD⁺ (regeneration of electron acceptors) for continuously performing the reaction.

The electron acceptors can be regenerated by adding enzymes that catalyze the regeneration reaction to the reaction system. For example, a method for regenerating NAD⁺ by adding diaphorase to the reaction system has been reported (J. Am. Chem. Soc. **107**, 6999-7008 (1985)). However, these conventional methods require expensive electron acceptors, coenzymes, enzymes for regenerating electron acceptors, and substrates for regeneration, and are thus economically disadvantageous.

It would be advantageous to efficiently regenerate electron acceptors concurrently with the oxidation reaction in microorganisms utilizing the electron acceptor regenerating system inherent in the microorganisms.

Many studies have been pursued so far on the method of culturing microorganisms for high level production of enzymes such as oxidoreductases. However, little is known for the method of culturing microorganisms for enhancing the activity to regenerate electron acceptors in the microorganisms.

In the culture of *Escherichia coli* into which a foreign oxidoreductase gene is introduced, high concentration of dissolved oxygen in the culture medium is maintained in order to increase the bacterial cell density by fed batch culture such as glucose feeding, or to suppress the production of acetic acid, a growth inhibitor. This method can increase the bacterial cell density and the oxidoreductase activity, but the microoragnsims show low activity to regenerate electron acceptors.

The present inventors prepared recombinant *Escherichia coli* capable of high level expression of secondary alcohol dehydrogenase that selectively acts on only the (S)-enatiomer of racemic 1,3-butanediol to oxidize alcohol at the 3-position and contacted the recombinant *Escherichia coli* thus obtained with racemic 1,3-butanediol to produce (R)-1,3-butanediol. However, although the amount of secondary alcohol dehydrogenase produced by the microorganism increased, its enzyme activity was not high enough to produce (R)-1,3-butanediol in a large quantity.

### SUMMARY OF THE INVENTION

An objective of the present invention is to enhance activity to regenerate electron acceptors for oxidoreductases in microorganisms capable of producing said oxidoreductases. Another objective of this invention is to provide a method for producing useful substances such as optically active alcohols using said microorganisms.

The present inventors already obtained *Escherichia coli* JM109 strain which produces the secondary alcohol dehydrogenase derived from *Candida parapsilosis*. This strain was prepared by the method described in Unexamined Published Japanese Patent Application No. (JP-A) Hei 7-231785. Using this *E. coli* strain, we studied effects of culture conditions on the activity to regenerate the oxidized form of the coenzyme.

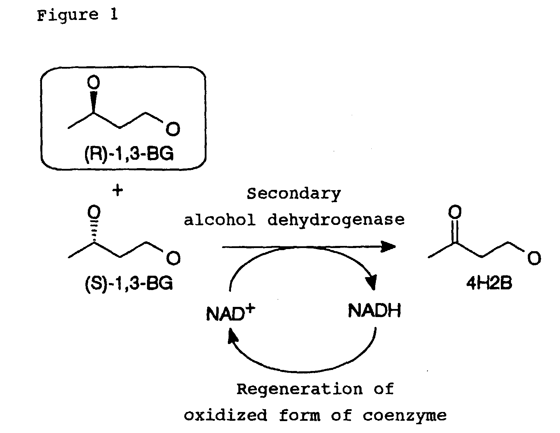

More specifically, we attempted to correlate culture conditions of the above-described *E. coli* capable of producing the secondary alcohol dehydrogenase with its activity to regenarate the oxidized form of the coenzyme in the synthetic process of (R)-1,3-butanediol (Fig. 1) in which the (S) enatiomer of racemic 1,3-butanediol (1,3-BG) is asymmetrically oxidized using the recombinant *E. coli*.

This process comprises oxidation reaction of (S)-1,3-butanediol catalyzed by the secondary alcohol dehydrogenase and regeneration reaction of NAD⁺ from NADH produced by the oxidation reaction. It is difficult to directly measure the activity to regenerate the oxidized form of the coenzyme. Therefore, while the activity to synthesize (R)-1,3-butanediol from racemic 1,3-butanediol by bacterial cells was assessed as the microbial cell activity, the activity to oxidize (S)-1,3-butanediol was measured using a disrupted bacterial cell suspension to assess the secondary alcohol dehydrogenase activity, thereby assessing the activity to regenerate the oxidized form of the coenzyme based on both of the microbial cell activity and the enzyme activity.

The microbial cell activity was assessed as follows. First, a given amount of cultured microbial cells were incubated with 5% 1,3-butanediol at 30°C for 17 h under shaking. The optical purity of 1,3-butanediol was measured, and the specific microbial activity was expressed as the percent change of optical purity per turbidity at 600 nm at the time of initiation of the reaction per one hour of the reaction time (ee%/OD600/hr). In addition, the microbial cell activity per culture medium was assessed using as a parameter the medium activity which is defined as the value obtained by multiplying the specific microbial activity by the turbidity of culture medium at 600 nm.

As a result, the present inventors discovered that the specific microbial activity was significantly enhanced by adjusting the concentration of dissolved oxygen in the culture medium of *E. coli* to a low level in spite of the low activity of secondary alcohol dehydrogenase, as compared with the activity obtained where the dissolved oxygen concentration was kept high,. The medium activity was also elevated under this condition.

The results that the bacterial cells had high activity to synthesize (R)-1,3-butanediol in spite of low secondary alcohol dehydrogenase activity can be interpreted that the culturing at low concentration of dissolved oxygen elevated activity to regenerate the oxidized form of the coenzyme in the bacterial cells.

This invention relates to a method for enhancing activity to regenerate an electron acceptor in microorganisms by adjusting the concentration of dissolved oxygen to a low level in the culture of microorganisms capable of producing oxidoreductase, and a method for efficient redox reaction using said microorganisms, and more specifically relates to:
(1) a method for enhancing activity to regenerate an electron acceptor for oxidoreductase of a microorganism capable of producing the enzyme, the method comprising culturing the microorganism in a culture medium with low concentration of dissolved oxygen during the period that the enzyme is expressed;
(2) the method according to (1), wherein the concentration of dissolved oxygen is 50% or less saturation;
(3) the method according to (1), wherein the concentration of dissolved oxygen is 20% or less saturation;
(4) the method according to (1), wherein the concentration of dissolved oxygen is 10% or less saturation;
(5) the method according to (1), wherein the oxidoreductase uses nicotinamide adenine dinucleotide (NAD⁺) or nicotinamide adenine dinucleotide phosphate (NADP⁺) as an electron acceptor;
(6) the method according to (1), wherein said oxidoreductase is alcohol dehydrogenase;
(7) the method according to (1), wherein said microorganism carries a foreign gene encoding oxidoreductase;
(8) the method according to (7), wherein said microorganism is *Escherichia coli*;
(9) a microorganism whose activity to regenerate an electron acceptor for oxidoreductase is enhanced by the method according to any one of (1) to (8);
(10) a method for producing an oxidized form of organic compound, the method comprising contacting the organic compound with the microorganism according to (9);
(11) the method according to (10), wherein the organic compound is alcohol; and
(12) a method for producing optically active alcohol, the method comprising contacting the microorganism according to (9) with racemic alcohol to specifically oxidize either (S)-enantiomer or (R)-enantiomer in the racemate.

### BRIEF DESCRIPTION OF THE DRAWINGS

Figure 1 is a diagram showing the process of synthesizing optically active alcohol.
Figure 2 shows graphs representing effects of dissolved oxygen concentrations on bacterial growth in batch culture.
Figure 3 shows graphs representing effects of dissolved oxygen concentrations on bacterial growth in fed-batch culture.

### DETAILED DESCRIPTION OF THE INVENTION

The term "alcohol" used herein means an organic compound having one or more hydroxyl groups and includes compounds such as aldehydes having hydroxyl group(s), amino acids having hydroxyl group(s), amino alcohols having hydroxyl group(s), amines having hydroxyl group(s), organic acids having hydroxyl group(s), hydroxyl carboxylic acids, and hydroxyl carboxylate esters.

This invention provides a method for enhancing activity of microorganisms to regenerate electron acceptors by culturing microorganisms capable of producing oxidoreductases at low concentration of dissolved oxygen during the period that the enzymes are expressed.

There is no particular limitation on microorganisms used in the method of this invention as long as they produce oxidoreductases. In addition, oxidoreductases and their sources are not particularly limited. Alcohol dehydrogenase is especially preferable. Secondary alcohol dehydrogenase derived from *Candida parapsilosis* is more preferable. The enzymes preferably requires NAD⁺ or NADP⁺ as an electron acceptor.

Microorganisms capable of producing oxidoreductases preferably express the enzymes of foreign origin. Such microorganisms include, for example, but not limited to, bacteria whose host-vector system have been developed such as genera *Escherichia*, *Bacillus*, *Pseudomonas*, *Serratia*, *Brevibacterium*, *Corynebacterium*, *Streptococcus*, *Lactobacillus*, etc.; yeasts such as genera *Saccharomyces*, *Kluyveromyces*, *Schizosaccharomyces*, *Zygosaccharomyces*, *Yarrowia*, *Trichosporon*, *Rhodosporidium*, *Hansenula*, *Pichia*, *Candida*, etc.; fungi belonging to genera *Neurospora*, *Aspergillus*, *Cephalosporium*, *Tricoderma*, etc. A preferable example is *Escherichia coli*.

Foreign oxidoreductases can be expressed in microorganisms by a standard method. For example, a microorganism (*E. coli* JM109) is transformed with a vector into which a gene encoding oxidoreductase (alcohol dehydrogenase) and allowed to express the enzyme as described in JP-A-Hei 7-231785.

The method of this invention comprises a step of culturing the microorganism in a medium containing low concentration of dissolved oxygen during the period that the enzyme is expressed, thereby elevating the activity of the microorganism to regenerate electron acceptors. The low concentration of dissolved oxygen means preferably 50% or less saturation, more preferably 20% or less saturation, and still more preferably 10% or less saturation (e.g. 0%) in the culture medium.

Both in a batch culture system and a fed batch culture system such as glucose-feeding, microorganisms with high activity to regenerate electron acceptors can be prepared by culturing the microorganisms in a culture medium having dissolved oxygen concentration of preferably 50% or less saturation, more preferably 20% or less saturation, and still more preferably 10% or less saturation (e.g. 0%).

The term "during the period that the enzyme is expressed" means, for example, after an inducer for expression of the enzyme is added to the culture medium where the enzyme is expressed by adding the inducer (inducible expression). When the enzyme is expressed without adding any inducer (constitutive expression) and when an inducer is added from the beginning of culturing of the microorganism (a kind of inducible expression), "the period that the enzyme is expressed" means the period from the logarithmic phase to the time of termination of culturing. During this period microorganisms actively proliferate and express oxidoreductase.

Culture conditions including a culture medium and a culturing temperature should be those preferable for the microorganism to be proliferated and to express oxidoreductase of the microorganism.

The concentration of dissolved oxygen in culture medium can be adjusted by automatically or manually varying, for example, the agitation rate, the aeration rate and/or pressure in the fermentor. In fed-batch culture, the concentration of dissolved oxygen in the culture medium can also be adjusted by varying the feeding rate of culture medium components containing the carbon source such as glucose, glycerol, sucrose, etc. to be fed during culturing. Alternatively, the concentration of dissolved oxygen can be adjusted by previously setting up the aeration rate, the agitation rate and pressure so that the concentration of dissolved oxygen in the culture medium becomes preferably 50% or less saturation, more preferably 20% or less saturation, and still more preferably 10% or less saturation (e.g. 0%) from the latter period to the time of termination of culturing. During this period the oxygen demand of the microorganisms is increased as they proliferate.

The concentration of dissolved oxygen in the culture medium can be measured by either the oxygen electrode method or the tubing method. Characteristics of each method are described in "Hakkokogaku no Kiso (Basic Fermentation Technology)" (Center for Academic Publications Japan, p158-159), etc. The oxygen electrode method is commonly used. There are two types of electrodes, galvanic and polarographic, and the polarographic electrode is generally used.

The timing for lowering the concentration of dissolved oxygen in the culture medium can be selected, by periodically measuring the enzyme activity, from the period from the time when the activity starts to increase to the time when the culture is terminated. It is necessary to reduce the concentration of dissolved oxygen at least one hour before termination of the culturing. For example, in the case of inducible expression, the timing to lower the dissolved oxygen concentration can be anytime during the period from the time after an inducer is added to one hour prior to the time of termination of culturing.

The time of termination of culturing can be determined by monitoring the cell density (determined by measuring the turbidity around 600 nm or the dry weight of microorganisms, etc.) and the specific microbial activity (oxidative activity per given amount of microbial cells). Specifically, culturing can be terminated at the time when the microbial oxidative activity per culture medium (not only oxidoreductase activity, but total oxidative activity of microbial cells including the activity to regenerate the coenzyme) becomes sufficiently high. The concentration of dissolved oxygen should be maintained low until the culturing is terminated.

When the concentration of dissolved oxygen in the culture medium is measured using a polarographic electrode, the concentration may temporarily shift to 50% or more saturation due to transient fluctuations in the agitation rate and the aeration rate. If the value quickly (e.g. in several seconds to several minutes) returns to the low level, such conditions are also included in the method of this invention for elevating the activity to regenerate electron acceptors for oxidoreductases.

Microorganisms prepared by the method of this invention characteristically have high activity to regenerate electron acceptors for the expressed oxidoreductases as compared with those cultured in the medium with usual dissolved oxygen concentrations (exceeding 50% saturation). The microorganisms have the high specific microbial activity and high medium activity if it has low oxidoreductase activity.

Microorganisms prepared by the method of this invention are capable of efficiently oxidizing organic compounds to produce corresponding oxidized forms through the redox reaction.

For example, as described in examples below, optically active alcohols can be produced by contacting the microorganisms with racemic alcohols to oxidize either (S)-enatiomer or (R)-enatiomer in the racemate.

The redox reaction can be performed by either in the culture medium of the microorganisms or in the reaction solution to which the microorganims isolated from the culture medium or treated products thereof are added. The treated microorganisms include microorganisms whose cell membrane permeability is modified by treating them with surfactants or organic solvents such as toluene, etc. The microbial cells can also be used by being immobilized on carageenan gel, alginate gel, polyacrylamide gel, cellulose, agar, or the like supporting material, using a known method.

The conditions suitable for the desired redox reaction, for example, the following conditions can be employed.
The reaction solution: an appropriate aqueous medium, or mixed system of an aqueous medium and an organic solvent,
The reaction temperature: 0 to 80°C (the high temperature within this range is used for thermophilic microorganisms, and the low temperature for psychrophilic microorganisms.),
The amount of microbial cells: culture medium containing microbial cells/the reaction solution = 0.01 /1 to 20/1 by volume, and
The substrate concentration: 0.01 to 90%.

Substrates can be supplied by continuously feeding or adding during the reaction.

The reaction products can be recovered from the microorganisms and the reaction solution by appropriate combinations of known techniques such as centrifugation, membrane treatment, solvent extraction, or distillation.

The products can be further purified, depending on characteristics of the products and remnants (substrates), by conventional methods, for example, chromatography such as ion-exchange chromatography and adsorption chromatography, extraction with a solvent, distillation, crystallization, etc. or in combination of these methods.

The present invention enables preparing microorganisms having high activity to regenerate the oxidized form of the coenzyme useful for the production of optically active alcohols, etc. by maintaining the concentration of dissolved oxygen in the culture medium at a low level during the period that the enzyme is expressed.

All publications, patent applications, patents, and other references mentioned herein are incorporated by reference.

In the following, this invention will be described in more detail with reference to examples, but is not to be construed being limited thereto.

### Example 1

### Preparation of E. coli expressing secondary alcohol dehydrogenase

Purification of secondary alcohol dehydrogenase derived from *Candida parapsilosis*, partial amino acid sequencing using the purified enzyme, designing of PCR primers based on the partial amino acid sequence, cloning of the gene encoding secondary alcohol dehydrogenase using the primers, and construction of the expression vector pKK-CPA1 using the cloned gene were performed by the methods described in JP-A-Hei 07-231785.

*E. coli* JM109 strain was transformed with the pKK-CPA1 thus constructed to obtain the recombinant *E. coli* JM109 (pKK-CPA1) expressing secondary alcohol dehydrogenase.

### Example 2

### Culturing of recombinant E. coli JM109 (pKK-CPA1) and inducible expression of the enzyme

Recombinant *E. coli* JM109 (pKK-CPA1) was inoculated into 5 ml of an LB medium (containing 10 g Bacto-Tryptone, 5 g Bacto-Yeast extract, and 10 g NaCl per liter) supplemented with 100 mg/l ampicillin, and cultured under shaking at 30°C for 12 h.

The culture medium thus obtained (600 µl) was inoculated into three 1.2-liter fermentors (Jars A, B, and C) (Marubishi Engineering) containing 600 ml each of 2 x YT medium (containing 20 g Bacto-Tryptone, 10 g Bacto-Yeast extract, and 10 g NaCl per liter) supplemented with 50 mg/l ampicillin, and cultured under aeration and shaking at 30°C and 540 rpm for about 9 h.

Turbidities of those culture media at 600 nm after the culturing were OD = 4.788 (Jar A, cultured for 9.1 h), OD = 4.385 (Jar B, after cultured for 9.0 h), and OD = 4.431 (Jar C, after cultured for 8.7 h).

20% lactose solution (67 ml) was added to each culture medium to induce the enzyme production. The agitation rate during the enzyme induction was set at 800 rpm for Jar A, 600 rpm for Jar B, and 400 rpm for Jar C. Seven hours after the initiation of enzyme induction, the culture media were sampled, and assayed for the enzyme activity, specific microbial activity and medium activity.

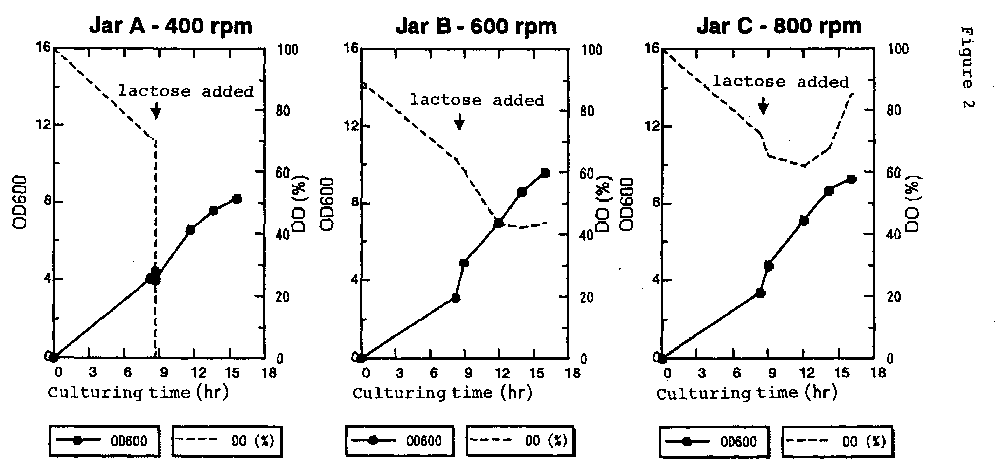

Turbidity at 600 nm and concentration of dissolved oxygen (DO, % saturation) of the culture medium during the culturing and enzyme induction are shown in Fig. 2.

### Example 3

### Assay of secondary alcohol dehydrogenase activity

Bacterial cells were collected by centrifuging 5 ml of the culture medium, suspended in 1.2 ml of buffer for cell disruption (consisting of 50 mM Tris-HCl buffer (pH 9.0), 0.02% 2-mercaptoethanol, and 2 mM phenylmethanesulfonyl chloride), and treated with a Mini-Bead beater (BIOSPEC) for 5 min to prepare cell-free extracts.

The secondary alcohol dehydrogenase activity was measured by reacting a reaction solution containing 50 mM Tris-HCl buffer (pH 9.0), 50 mM (S)-1,3-butanediol, and 2.5 mM nicotinamide adenine dinucleotide (NAD⁺) at 30°C and measuring the increase in absorption at 340 nm due to the generation of the reduced nicotinamide adenine dinucleotide (NADH). One unit (1 U) of enzyme activity was defined as the amount of enzyme to produce 1 µmol of NADH per min.

The results are shown in Table 1. In the table, the enzyme activity is represented as the activity per 1 ml of the culture medium (broth) (U/ml-br) and that per turbidity at 600 nm (U/OD). The enzyme activity was highest when the agitation rate was set at 600 rpm during the enzyme induction.

### Example 4

### Assay of specific microbial activity

Culture medium corresponding to the turbidity at 600 nm (OD600) of 40 was centrifuged to prepare wet cells. The cells were suspended in 25 ml of a reaction solution containing 5% 1,3-butanediol (racemate), incubated at 30°C for 17 h under shaking. The optical purity of 1,3-butanediol remaining in the reaction solution was measured.

The specific microbial activity was expressed as the percent change of optical purity per turbidity of bacterial cells (turbidity at 600 nm) per one hour of the reaction time (%/OD/hr).

The optical purity of 1,3-butanediol remaining in the reaction solution was measured by extracting 1,3-butanediol with ethyl acetate from the reaction solution, evaporating the extract to dryness, acetylating the resulting residue with acetyl chloride, and fractionating the acetylated product on a Chiralcel OB column (Daicel Chemical Industries, Ltd.). Elution was performd with n-hexane:isopropanol = 19:1 v/v as an eluent, and the absorbance at 220 nm of eluates was determined with an UV detector.

The results are shown in Table 1. While the cells cultured at 600 rpm showed the highest enzyme activity, the cells cultured at 400 rpm showed the lowest DO (approximately DO = 0% saturation during the enzyme induction) and the highest specific microbial activity.

In addition, the medium activity obtained by multiplying the specific microbial activity by the OD600 value of the culture medium is also shown in Table 1 as a factor representing the productivity per medium.

The cells cultured at 400 rpm exhibited low OD of the culture medium, while the specific microbial activity and medium activity were highest. The results indicate that these conditions (approximately DO = 0% during the enzyme induction) were most suitable for the purpose of this invention.

**Table 1**

| Agitation conditions in batch culture and activities | | | | | |
|---|---|---|---|---|---|
| Agitation rate (rpm) | OD600 | Enzyme activity | | Specific microbial activity | Medium activity |
| | | U/ml-br | U/OD | | |
| 400 | 8.211 | 6.77 | 0.825 | 1.790 | 14.7 |
| 600 | 9.618 | 12.05 | 1.253 | 0.864 | 8.31 |
| 800 | 9.303 | 9.84 | 1.058 | 0.852 | 7.93 |

### Example 5

### Effects of dissolved oxygen concentration in fed-batch culture

The recombinant *E. coli* JM109 (pKK-CPA1) strain was inoculated into 50 ml of an LB medium (10 g Bacto-Tryptone, 5 g Bacto-Yeast extract, and 10 g NaCl in 1 liter) supplemented with 50 mg/l ampicillin, and cultured under shaking at 25°C for 23 h in the same manner as in Example 2.

The culture medium thus obtained (6 ml) was inoculated into a 1.2-liter fermentor (Marubishi Engineering) containing 600 ml of a fed-batch culture medium (15.14 g Na₂HPO₄·12H₂O, 3.0 g KH₂PO₄, 5.0 g NaCl, 2.0 g NH₄Cl, 5.0 g Peptone, 25.0 g glucose, 0.24 g MgSO₄·7H₂O, 0.99 mg FeSO₄·7H₂O, 0.88 mg ZnSO₄·7H₂, 0.393 mg CuSO₄·7H₂O, 0.072 mg MnCl₂·4H₂O, 0.088 mg Na₂B₄O₇·10H₂O, 0.037 mg (NH₄)₆Mo₇O₂₄·4H₂O, and 4.0 mg thiamine in 1 liter).

The bacteria were cultured at 700 rpm, with the aeration rate of 0.6 l/min at 30°C. After about 17 h, glucose in the medium was exhausted, and the continuous glucose feeding at a constant rate was initiated. In addition, after the initiation of glucose feeding, the concentration of dissolved oxygen was manually adjusted to 10% or less saturation (in Jar A) and 20% or less saturation (in Jar B) by varying the agitation rate.

Twenty-three hours after the initiation of culturing, 2% lactose was added to the medium to induce the enzyme production. The dissolved oxygen concentration was continuously adjusted during the enzyme induction.

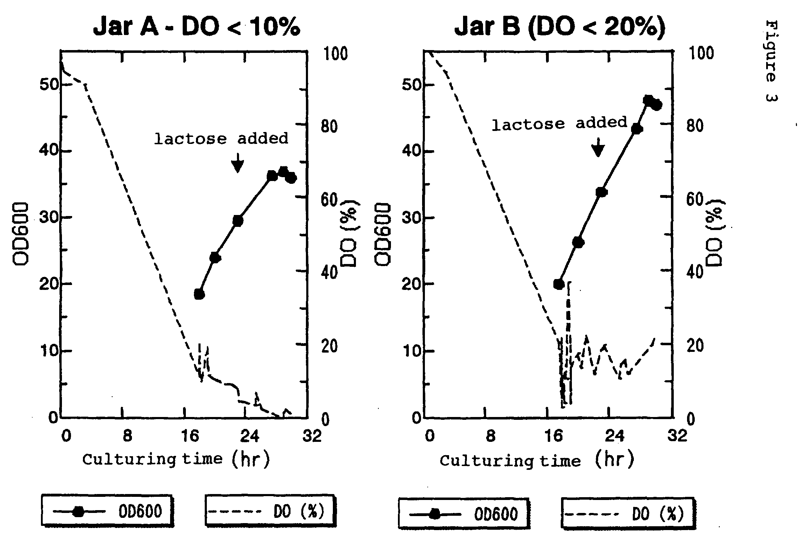

After 30-h culturing, the culture solution was sampled and assayed for the secondary alcohol dehydrogenase activity and microbial cell activity. Turbidity at 600 nm (OD600) and dissolved oxygen concentration (DO (%)) during the culturing are shown in Fig. 3.

### Example 6

### Activity of bacterial cells obtained by fed-batch culture

The secondary alcohol dehydrogenase activity, specific microbial activity and medium activity of the cells obtained in Example 5 were assayed by the methods described in Examples 3 and 4. The results are shown in Table 2.

As compared with the cells cultured at the dissolved oxygen concentration of approximately 20% or less saturation, the cells cultured at the dissolved oxygen concentration of approximately 10% or less saturation showed lower enzyme activity and turbidity, but significantly high specific microbial activity and excellent medium activity. These results indicate that microorganisms having higher activity to regenerate the oxidized form of the coenzyme could be prepared by adjusting the dissolved oxygen concentration of culture medium 10% or less saturation.

**Table 2**

| Activities of bacterial cells prepared by fed-batch culture | | | | | |
|---|---|---|---|---|---|
| DO(%) | OD600 | Enzyme activity | | Specific microbial activity | Medium activity |
| | | U/ml-br | U/OD | | |
| <10 | 36.05 | 31.8 | 0.881 | 1.36 | 49.2 |
| <20 | 47.06 | 64.0 | 1.360 | 0.98 | 46.2 |

## Claims

1. A method for enhancing activity to regenerate an electron acceptor for oxidoreductase of a microorganism capable of producing the enzyme, the method comprising culturing the microorganism in a culture medium with low concentration of dissolved oxygen during the period that the enzyme is expressed.

2. The method according to claim 1, wherein the concentration of dissolved oxygen is 50% or less saturation.

3. The method according to claim 2, wherein the concentration of dissolved oxygen is 20% or less saturation.

4. The method according to claim 3, wherein the concentration of dissolved oxygen is 10% or less saturation.

5. The method according to any one of claims 1 to 4, wherein the oxidoreductase uses nicotinamide adenine dinucleotide (NAD⁺) or nicotinamide adenine dinucleotide phosphate (NADP⁺) as an electron acceptor.

6. The method according to any one of claims 1 to 5, wherein said oxidoreductase is alcohol dehydrogenase.

7. The method according to any one of claims 1 to 6, wherein said microorganism carries a foreign gene encoding oxidoreductase.

8. The method according to any one of claims 1 to 7, wherein said microorganism is selected from the group consisting of *Escherichia*, *Bacillus*, *Pseudomonas*, *Serratia*, *Brevibacterium*, *Corynebacterium*, *Streptococcus*, *Lactobacillus*, *Saccharomyces*, *Kluyveromyces*, *Schizosaccharomyces*, *Zygosaccharomyces*, *Yarrowia*, *Trichosporon*, *Rhodosporidium*, *Hansenula*, *Pichia*, *Candida*, *Neurospora*, *Aspergillus, Cephalosporium,* and *Tricoderma*.

9. The method according to claim 8, wherein said microorganism is *Escherichia coli*.

10. A microorganism capable of producing oxidoreductase whose activity to regenerate an electron acceptor for oxidoreductase is enhanced by the method according to any one of claims 1 to 9.

11. A method for producing an oxide of an organic compound, the method comprising contacting the organic compound with the microorganism of claim 10.

12. The method according to claim 11, wherein the organic compound is alcohol.

13. A method for producing optically active alcohol, the method comprising contacting the microorganism of claim 10 with racemic alcohol to specifically oxidize either (S)-enantiomer or (R)-enantiomer in the racemate.
